(19) **Europäisches Patentamt**

**European Patent Office**

**Office européen des brevets**

(11) Veröffentlichungsnummer : **0 335 825 B1**

(12) # EUROPÄISCHE PATENTSCHRIFT

(45) Veröffentlichungstag der Patentschrift :
**02.09.92 Patentblatt 92/36**

(21) Anmeldenummer : **89730065.3**

(22) Anmeldetag : **10.03.89**

(51) Int. Cl.⁵ : **C02F 3/28,** C12M 1/00,
**C05F 3/06**

(54) **Verfahren und Vorrichtung zur zweistufigen anaeroben Aufbereitung flüssiger Substrate.**

(30) Priorität : **23.03.88 DE 3810250**

(43) Veröffentlichungstag der Anmeldung :
**04.10.89 Patentblatt 89/40**

(45) Bekanntmachung des Hinweises auf die
Patenterteilung :
**02.09.92 Patentblatt 92/36**

(84) Benannte Vertragsstaaten :
**AT BE CH DE ES GB IT LI LU NL**

(56) Entgegenhaltungen :
**EP-A- 0 118 621**
**EP-A- 0 172 443**
**EP-A- 0 193 969**
**DE-A- 581 860**
**DE-A- 3 102 739**
**DE-A- 3 214 798**

(56) Entgegenhaltungen :
**DE-A- 3 228 782**
**DE-A- 3 530 332**
**DE-A- 3 623 431**
**GB-A- 2 013 170**

(73) Patentinhaber : **MANNESMANN**
**Aktiengesellschaft**
**Mannesmannufer 2**
**W-4000 Düsseldorf 1 (DE)**

(72) Erfinder : **Bracker, Gerd-Peter, Dr.-Ing.**
**Weidengrund 18**
**W-4630 Bochum 1 (DE)**
Erfinder : **Scholz, Werner, Dr. rer. nat.**
**Lilienweg 4**
**W-4670 Lünen (DE)**

(74) Vertreter : **Meissner, Peter E., Dipl.-Ing. et al**
**Patentanwaltsbüro Meissner & Meissner,**
**Herbertstrasse 22**
**W-1000 Berlin 33 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur zweistufigen anaeroben Aufbereitung flüssiger Substrate mit hoher organischer Belastung gemäß dem Oberbegriff des Patentanspruchs 1 sowie eine Vorrichtung zur Durchführung des Verfahrens. Als flüssiges Substrat in diesem Sinne sind Abwässer, Gülle und ähnliche flüssige Stoffgemische mit organischem Feststoffanteil anzusehen.

Die Zweistufigkeit der anaeroben Aufbereitung von Substraten ist der Natur nachempfunden. In der ersten Stufe werden die makromolekularen Verbindungen zunächst hydrolysiert. Die Organismengruppe, die in dieser Stufe tätig ist, spaltet die hochmolekularen Verbindungen wie Stärke, Proteine und Fette in niedermolekulare Substanzen wie Zucker, Aminosäuren, Glyzerin und Fettsäuren. Die Hydrolyse der Biopolymere erfolgt sehr schnell mit Ausnahme von lignocellulosehaltigen Materialien, da das Lignin anaerob nur sehr langsam gespalten werden kann.

Die entstandenen Hydrolyseprodukte werden mit Ausnahme der Fettsäuren weiter mikrobiell bei einem für diesen Vorgang günstigen pH-Wert unter 6 zu Wasserstoff, Kohlendioxid, Carbonsäuren, Alkoholen und anderen ähnlichen Verbindungen umgesetzt.

In der nachfolgenden zweiten Stufe, die am günstigsten bei einem pH-Wert um 7 stattfindet, werden diese Verbindungen durch bestimmte Bakterien zu Essigsäure, Wasserstoff und Kohlendioxid umgesetzt. Diese Bakteriengruppe kann nur bei einem sehr niedrigen Wasserstoffpartialdruck wachsen. Sie lebt bei einem pH-Wert um 7 in Symbiose mit den sauerstoffempfindlichen methanbildenden Bakterien, die auch den Wasserstoff umsetzen. Der Wasserstofftransfer von den essigsäure- und wasserstoffbildenden Bakterien zu den Methanbakterien ist häufig der bestimmende Faktor bei der mikrobiellen Methanbildung.

Ein derartiges Verfahren ist aus der deutschen Patentschrift 32 03 445 bekannt. Im Hinblick auf die Anlagentechnik sieht dieses Verfahren eine getrennte Anordnung des Reaktors für die Hydrolyse und die saure Gärung einerseits und des für die Methanfaulung andererseits vor.

Weiterhin ist aus den deutschen Patentschriften 31 02 739 und 32 14 798 ein Verfahren bekannt, in dem beide Stufen in den getrennten Kammern eines Doppelkammerreaktors ablaufen. Hierbei wird von einer volumenmäßigen Aufteilung entsprechend dem zugrundegelegten Zeitbedarf der Säurephase und der Methanphase im Verhältnis von 1 : 10 ausgegangen. Die Aufteilung des Reaktors ist in Form von zwei konzentrischen Kammern, die im Bodenbereich miteinander verbunden sind, realisiert. Nachteilig ist hierbei, daß bei dieser Anordnung ein Umwälzen der Flüssigkeiten im Faulraum nicht möglich ist und daß der Gärreaktor von oben beschickt wird. Letzteres ist ungünstig im Hinblick auf die erwünschte möglichst weitgehende Absetzung von Feststoffen im Gärreaktor, die für den Zeitbedarf der nachfolgenden Methanfaulung bestimmend ist.

Aus der DE-A-32 28 782 ist eine Vorrichtung-zur anaeroben Behandlung von Schlämmen und Abwässern bekannt, bei der in den zylindrischen Behälter ein Reaktionsraum zur Durchführung der Methangärung und eine Trennkammer vorgesehen sind, in der Trenneffekte durch Sedimentation und Flotation genutzt werden.

Die den Reaktionsraum von der Trennkammer trennende Wand weist in ihrem unteren Teil Durchtrittsöffnungen auf und unterhalb diesen ist in der Trennkammer ein Sammelraum für den eingedickten Schlamm ausgebildet.

Schließlich ist noch aus der DE-A-36 23 431 ein mehrstufiger Bioreaktor bekannt, bei dem - ebenso wie bei der vorher behandelten Vorrichtung - nur ein einziger Schlammabzug vorgesehen ist, und zwar in diesem Fall von der Gärkammer. Die Gärkammer ist dabei in zwei Teilstufen unterteilt, in denen unterschiedliche Strömungen eingestellt werden.

Der Erfindung liegt die Aufgabe zugrunde, ein Verfahren der gattungsgemäßen Art in der Weise weiterzubilden, daß der Gesamtprozeß der Substrataufbereitung beschleunigt wird. Ferner soll eine Vorrichtung zur Durchführung dieses Verfahrens angegeben werden, die möglichst einfach aufgebaut ist und hinsichtlich der optimalen Größen der Gär- und Faulkammer leicht an bestehende Verhältnisse, d.h. an den Grad der organischen Belastung des zu behandelnden Substrates angepaßt werden kann.

Diese Aufgabe wird hinsichtlich des Verfahrens erfindungsgemäß durch die kennzeichnenden Merkmale des Patentanspruchs 1 gelöst; vorteilhafte Weiterbildungen des Verfahrens sind in den Unteransprüchen 2 - 6 angegeben.

Die Merkmale einer erfindungsgemäßen Vorrichtung zur Durchführung des Verfahrens ergeben sich aus dem Patentanspruch 7; vorteilhafte Weiterbildungen dieser Vorrichtung sind in den Unteransprüchen 8 - 17 gekennzeichnet.

Wesentliches Merkmal der Erfindung ist, daß die Durchleitung des zu behandelnden Substrates durch die beiden Kammern des Reaktors nach dem Aufstromprinzip erfolgt. Hierzu wird das Substrat der Gärkammer über eine Zuleitung zugeführt, die etwa auf einem Drittel der Höhe des Flüssigkeitsspiegels in der Gärkammer oder darunter in diese mündet. In dieser ersten Kammer erfolgen die Hydrolyse und die saure Gärung. Der Gärschlamm setzt sich infolge des angewandten Aufstromverfahrens besonders effektiv ab und kann nach dem Abziehen durch Waschen oder Kompostieren nachbehandelt werden. Das entstehende Gärgas ($H_2$, $CO_2$, $H_2S$) strömt am Reaktorkopf aus.

Die von organischen Feststoffen bereits weitgehend befreite Flüssigkeit fließt frei über in die zweite Kammer und wird dabei durch eine Leitung so geführt, daß sie wiederum etwa auf einem Drittel der Höhe des Flüssigkeitsspiegels oder darunter in diese Kammer eintritt, in der die acetogene Phase und die Methanbildung stattfinden. Zur Beschleunigung des Faulprozesses ist es sehr vorteilhaft, die Flüssigkeit zwangsweise umzuwälzen, um so ein Bakterienschlammbett durch Agglomeration aufzubauen. Sich absetzender Faulschlamm wird am Boden bei Bedarf abgezogen. Das sich bei der Faulung bildende Biogas ($CH_4$ und $CO_2$) verläßt den Reaktor am Kopf. Das frei überlaufende Faulwasser kann vor seiner Abgabe in die Kanalisation gemäß den Einleitungsbedingungen z.B. aerob nachbehandelt werden. Die Verweilzeit der Flüssigkeit in den Reaktorkammern richtet sich nach dem Grad der organischen Belastung und nach der Hydrolyse- und Gärungsgeschwindigkeit der organischen Stoffe, wobei die erste Stufe 1,5- bis 5-mal schneller abläuft als die zweite. Prinzipiell gilt, je mehr organische Feststoffe im Substrat enthalten sind, umso länger dauert der Faulprozeß, d.h. umso kleiner wird das Verhältnis der Volumina von Gärkammer und Faulkammer.

Letztlich ist dieses Verhältnis selbstverständlich vom gewünschten Grad der Aufbereitung des Substrates abhängig, was sich exakt in der Differenz der Anfangs- und Endwerte für den chemischen und biologischen Sauerstoffbedarf (CSB bzw. $BSB_5$) ausdrücken läßt. Für die meisten Anwendungsfälle, bei denen ein relativ feststoffarmes Substrat zu behandeln ist, kann ein Verhältnis von 1 : 2 als günstig angesehen werden. Der Reinigungsprozeß kann sowohl mesophil als auch thermophil durchgeführt werden.

Eine Verbesserung der Effektivität des Faulprozesses läßt sich dadurch erreichen, daß in der Faulkammer eine Schicht aus einem porösen, flüssigkeitsdurchlässigen Feststoff mit großer spezifischer Oberfläche angeordnet wird. Auf der Oberfläche eines solchen "Filterkörpers" können sich Mikroorganismen in großer Zahl ansiedeln. Damit kann eine hohe Bakteriendichte gewährleistet werden, wobei die Gefahr des Ausschwemmens dieser Bakterien mit dem Abzug des gereinigten Abwassers minimiert ist. Um eine Verstopfung des "Filterkörpers", der z.B. als Schüttung von Feststoffteilchen auf einem Siebboden ausgebildet sein kann, zu vermeiden, ist es zweckmäßig, die Förderrichtung der Zwangsumwälzung zur Erzielung eines Rückspüleffektes zeitweilig umzukehren.

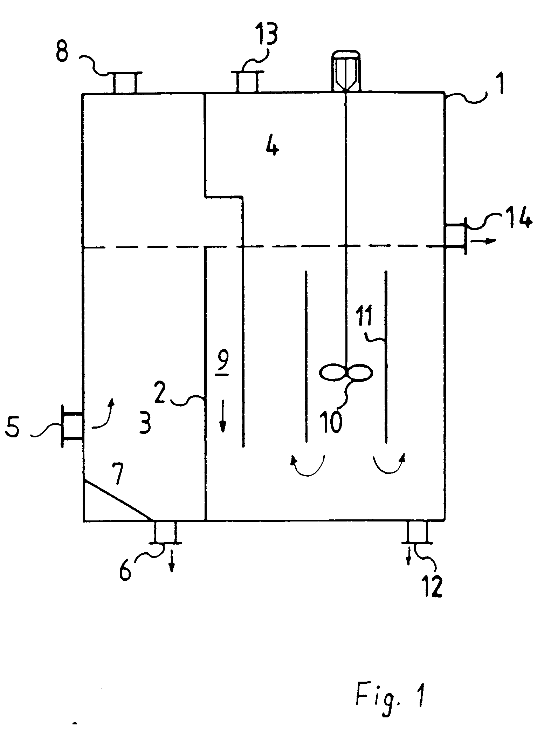

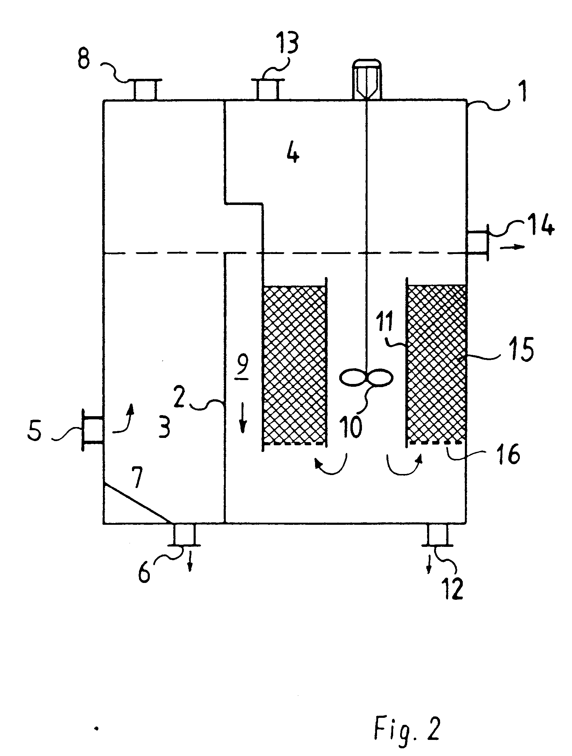

Anhand der in der Figuren 1 und 2 dargestellten Ausführungsbeispiele eines erfindungsgemäßen Doppelkammerreaktors wird die Erfindung im folgenden näher erläutert.

Zur Durchführung des Verfahrens eignet sich, wie in Fig. 1 dargestellt, ein runder oder auch eckiger Behälter 1 mit einer Trennwand 2, die den Behälter 1 in zwei Kammern 3, 4 unterteilt. Die kleinere Kammer dient als Gärkammer 3 und die größere als Faulkammer 4. Zwischen den beiden Kammern 3, 4 besteht eine Verbindung in Form eines rohrartigen überlaufs 9, der innerhalb der Faulkammer 4 vertikal nach unten geführt ist. In gleicher Höhe wie der Überlauf 9 ist in der Wand des Behälters 1 ein Anschluß 14 für die Abwasserableitung aus der Faulkammer 4 angeordnet. Der Flüssigkeitsspiegel in den beiden Kammern 3, 4 ist daher gleich hoch.

Die Zuführung des zu reinigenden Substrates zur Gärkammer 3 erfolgt über einen im unteren Drittel des Flüssigkeitsspiegels mit Abstand vom Reaktorboden in der äußeren Wand des Behälters 1 angebrachten Anschluß 5, so daß das Substrat nach dem vorteilhaften Aufstromprinzip eingeleitet wird und eine weitgehende Befreiung von den darin enthaltenen Feststoffen stattfinden kann, bevor die nach der Hydrolyse und der sauren Gärung verbleibende Flüssigkeit in die Faulkammer 4 eintritt.

Die Feststoffe setzen sich am Boden der Gärkammer 3, die vorteilhaft mit einem Schrägboden 7 ausgestattet ist, ab, so daß der sedimentierte Gärschlamm durch den Abflußstutzen 6 im Behälterboden abgezogen werden kann. Das entstehende Gärgas sammelt sich oberhalb des Flüssigkeitsspiegels in der Gärkammer 3 und wird über den Anschlußstutzen 8 abgeleitet.

Der Überlauf 9 mündet im Abstand vom Reaktorboden etwa auf einem Drittel der Höhe des Flüssigkeitsspiegels in die Faulkammer 4, so daß auch die Durchleitung des vorbehandelten Substrates durch die Faulkammer 4 nach dem Aufstromprinzip erfolgt. Um den Faulprozeß zu intensivieren ist es sehr zweckmäßig, in der Faulkammer 4 eine Zwangsumwälzung zu gewährleisten. Diese ist gemäß der Figur als Rührer 10 ausgeführt, der in einem rohrförmigen Mantel 11 angeordnet ist, damit eine sichere, gezielte Umwälzung gegeben ist. Der Rührer 10 und der Mantel 11 werden zweckmäßigerweise so ausgelegt, daß außerhalb des rohrförmigen Mantels 11 eine Aufströmgeschwindigkeit von 0,8 bis 1,3 m/h herrscht, um eine größtmögliche Bakteriendichte zu erhalten. Bevorzugt wird die Förderrichtung des Rührers 10 daher auf Abwärtsförderung eingestellt.

Da das Substrat in der Gärkammer 3 bereits weitgehend von Feststoffen befreit wurde, entsteht in der Faulkammer 4 nur noch wenig Faulschlamm, der zum Teil am Boden sedimentiert, wo er durch den Stutzen 12 bei Bedarf problemlos abgezogen werden kann. Das sich bildende Faulgas verläßt den Faulbehälter 4 in vorteil-

3

hafter Weise getrennt vom Gärgas durch den Stutzen 13 am Kopf des Reaktors 1. Die ausgefaulte Flüssigkeit läuft durch den Stutzen 14 als Abwasser ab.

Bei einer bevorzugten Ausführung des erfindungsgemäßen Reaktors liegen Zuführstutzen 5 und Abführstutzen 14 dicht beieinander und sind in einen Wärmetauscher in der Weise eingebunden, daß die mit dem Abwasser aus dem Behälter 1 herausgehende Wärmeenergie für die Vorwärmung des frisch zugeführten Substrates benutzt werden kann.

Die in Fig. 2 dargestellte bevorzugte Ausführungsform des erfindungsgemäßen Reaktors unterscheidet sich von Fig. 1 lediglich dadurch, daß um den Mantel 11 der Rührvorrichtung 10 herum eine auf einem Siebboden 16 ruhende Schüttung 15 von Feststoffteilchen mit poröser Oberfläche angeordnet ist. Auf der porösen Oberfläche der z.B. aus Keramik bestehenden Feststoffteilchen siedeln sich Mikroorganismen an, die nicht mehr so leicht aus der Faulkammer 4 über den Abwasserstutzen 14 ausgeschwemmt werden, sondern den Faulprozeß intensivieren. Um eine Verstopfung der wie ein Filterkörper wirkenden Feststoffschüttung 15 zu vermeiden, sollte die Förderrichtung der Rührvorrichtung 10 zeitweilig umgekehrt werden, so daß der Rückspüleffekt zu einer ausreichenden Befreiung der Schüttung 15 von organischen Feststoffen führt.

Besondere Vorteile des erfindungsgemäßen Reaktors liegen neben einer Beschleunigung des gesamten Prozesses darin, daß

– ein Zwischenaufheizen wie bei einer Anlage mit zwei getrennten Reaktoren nicht erforderlich ist,
– die Wärmeverluste durch die kompakte Bauweise insgesamt minimiert werden,
– nur eine Beschickungspumpe benötigt wird und
– eine sehr einfache Anpassung an die Erfordernisse der jeweils vorliegenden Substratbelastung bzw. des zu erzielenden Reinigungsgrades erfolgen kann, indem durch ein einfaches Versetzen der Trennwand ein optimales Verhältnis der Kammervolumina eingestellt wird.

## Patentansprüche

1. Verfahren zur zweistufigen anaeroben Aufbereitung flüssiger Substrate mit hoher organischer Belastung in einem einzigen Reaktor mit zwei durch eine Trennwand voneinander getrennten Kammern, in deren erster die Hydrolyse und die saure Gärung (Gärkammer) und in deren zweiter die Methanbildung (Faulkammer) stattfindet, wobei sedimentierte Feststoffe im Bodenbereich der Gärkammer abgezogen werden, wobei ferner die Einleitung des Substrates aus dem oberen Bereich der Gärkammer heraus in die Faulkammer erfolgt, wobei außerdem der Abwasseranteil des behandelten Substrates oben aus der Faulkammer abgeleitet wird und wobei das gebildete Gärgas und das Faulgas oberhalb des Flüssigkeitsspiegels abgezogen werden,
   dadurch gekennzeichnet,
   daß das Substrat der Gärkammer im Abstand vom Boden der Gärkammer in einer Höhe von maximal einem Drittel des Flüssigkeitsspiegels der Gärkammer zugeführt wird, daß die Einleitung des von Feststoffen weitgehend befreiten Substrats in die Faulkammer im Abstand vom Boden der Faulkammer in einer Höhe von maximal einem Drittel des Flüssigkeitsspiegels der Faulkammer erfolgt, daß sedimentierte Feststoffe aus der Faulkammer abgezogen werden und daß das Substrat in der Faulkammer zwangsumgewälzt wird.

2. Verfahren nach Anspruch 1,
   dadurch gekennzeichnet,
   daß die Aufströmgeschwindigkeit in der Faulkammer im Bereich 0,8 bis 1,3 m/h gehalten wird.

3. Verfahren nach einem der Ansprüche 1 - 2,
   dadurch gekennzeichnet,
   daß die Abwasserbehandlung in einem kontinuierlichen Betrieb erfolgt.

4. Verfahren nach einem der Ansprüche 1 - 3,
   dadurch gekennzeichnet,
   daß das Gärgas und das Faulgas getrennt voneinander erfaßt und abgezogen werden.

5. Verfahren nach einem der Ansprüche 1 - 4,
   dadurch gekennzeichnet,
   daß das Substrat innerhalb der Faulkammer durch eine Schüttung aus Feststoffteilchen, auf deren Oberfläche Mikroorganismen angesiedelt sind, hindurchgeführt wird.

6. Verfahren nach Anspruch 5,
   dadurch gekennzeichnet,
   daß die Strömungsrichtung des Substrates in der Faulkammer zeitweilig umgekehrt wird.

7. Bioreaktor zur Durchführung des Verfahrens nach Anspruch 1, bestehend aus einem Behälter (1) mit einer ersten Kammer (Gärkammer 3) für die Hydrolyse und die saure Gärung und einer durch eine Zwischenwand (2) abgetrennten zweiten Kammer (Faulkammer 4) für die Methanbildung, mit einer Zuleitung (5) für ein flüssiges Substrat zur Gärkammer (3) und einer Ableitung (14) für das behandelte Substrat aus der Faulkammer (4), mit einer Gasableitung (8, 13) für das entstehende Gärgas und Faulgas und mit einer Abzugsvorrichtung (6) für den im Bodenbereich der Gärkammer (3) sich bildenden Schlamm,
   dadurch gekennzeichnet,
   daß die Substratzuleitung (5) in die Gärkammer (3) im Abstand vom Boden der Gärkammer (3) höchstens auf einem Drittel der vorgesehenen Höhe des Flüssigkeitsspiegels in der Gärkammer (3) angeordnet ist, daß die Verbindung zwischen der Gärkammer (3) und der Faulkammer (4) als Überlauf (9) gestaltet ist, der in Form einer nach unten geführten Rohrleitung bis mindestens etwa auf ein Drittel der Höhe der ebenfalls als Überlauf ausgebildeten Abwasserableitung (14) in die Faulkammer (4) hinunterreicht und im Abstand vom Boden der Faulkammer (4) endet und daß in der Faulkammer (4) eine Zwangsumwälzvorrichtung sowie ein Abflußstutzen (12) im Boden für den Faulschlamm vorgesehen sind.

8. Bioreaktor nach Anspruch 7,
   dadurch gekennzeichnet,
   daß die Zwangsumwälzvorrichtung als Rührvorrichtung (10) mit einem diese umgebenden vertikal gerichteten rohrförmigen Mantel (11) ausgebildet ist, der jeweils im Abstand vom Reaktorboden und vom Flüssigkeitsspiegel endet.

9. Bioreaktor nach einem der Ansprüche 7 - 8,
   dadurch gekennzeichnet,
   daß die die beiden Kammern (3, 4) voneinander abtrennende Zwischenwand (2) in der Weise angeordnet ist, daß das Volumenverhältnis der Gärkammer (3) zur Faulkammer (4) zwischen 1 : 1,5 und 1 : 5 beträgt.

10. Bioreaktor nach einem der Ansprüche 7 - 9,
    dadurch gekennzeichnet,
    daß die Zwangsumwälzvorrichtung auf eine Aufströmgeschwindigkeit im Bereich 0,8 bis 1,3 m/h ausgelegt ist.

11. Bioreaktor nach einem der Ansprüche 7 - 10,
    dadurch gekennzeichnet,
    daß die Substratzuleitung (5) und die Abwasserableitung (14) dicht benachbart aus der Wand des Reaktors (1) herausgeführt und in die Wärmeabgabe- bzw. Wärmeaufnahmeseite eines Wärmetauschers integriert sind.

12. Bioreaktor nach einem der Ansprüche 7 - 11,
    dadurch gekennzeichnet,
    daß die Gärkammer (3) und die Faulkammer (4) jeweils eine separate Gasableitung (8 bzw. 13) aufweisen.

13. Bioreaktor nach einem der Ansprüche 7 - 12,
    dadurch gekennzeichnet,
    daß in der Faulkammer (4) oberhalb der Mündung des überlaufs 9 eine Schicht aus einer Schüttung (15) von Feststoffteilchen mit poröser Oberfläche angeordnet ist.

14. Bioreaktor nach Anspruch 13,
    dadurch gekennzeichnet,
    daß die Feststoffteilchen aus Keramik gebildet sind.

15. Bioreaktor nach Anspruch 13 oder 14,
    dadurch gekennzeichnet,
    daß die Schüttung (15) auf einem als Siebboden (16) ausgebildeten Zwischenboden angeordnet ist.

16. Bioreaktor nach einem der Ansprüche 13 - 15,
    dadurch gekennzeichnet,
    daß die Schüttung (15) sich über den gesamten Querschnitt der Faulkammer (4) erstreckt.

17. Bioreaktor nach Anspruch 10 und einem der Ansprüche 13 - 16,
    dadurch gekennzeichnet,
    daß die Schüttung (15) um den rohrförmigen Mantel (11) der Rührvorrichtung (10) herum angeordnet ist.


**Claims**

1. A method for the two-stage anaerobic treatment of liquid substrates with a high organic load in a single reactor having two chambers separated from one another by a partition, in the first of which the hydrolysis and the acid fermentation (fermentation chamber) and in the second of which the methane formation (digestion chamber) takes place, with sedimented solids being drawn off in the bottom region of the fermentation chamber, furthermore the introduction of the substrate taking place out of the upper region of the fermentation chamber and into the digestion chamber, furthermore the content of waste water of the treated substrate being diverted from the digestion chamber at the top and the resulting fermentation gas and the digester gas being drawn off above the level of the liquid,
   characterised in that
   the substrate of the fermentation chamber is supplied at a distance from the bottom of the fermentation chamber at a height of at most one third of the liquid level of the fermentation chamber, that the introduction of the substrate which is largely freed of solids into the digestion chamber takes place at,a distance from the bottom of the digestion chamber at a height of at most one third of the liquid level of the digestion chamber, that sedimented solids are drawn off out of the digestion chamber and that the substrate is subjected to forced circulation in the digestion chamber.

2. A method according to Claim 1, characterised in that the upward stream rate inthe digestion chamber is kept in the range of 0.8 to 1.3 m/h.

3. A method according to one of Claims 1 - 2, characterised in that the treatment of waste water takes place in a continuous operation.

4. A method according to one of Claims 1 - 3, characterised in that the fermentation gas and the digester gas are gathered and drawn off separately from one another.

5. A method according to one of Claims 1 - 4, characterised in that the substrate within the digestion chamber is passed through a bed of solids particles, the surface of which has been colonised by microorganisms.

6. A method according to Claim 5, characterised in that the direction of flow of the substrate in the digestion chamber is reversed temporarily.

7. A bioreactor for performing the method according to Claim 1, consisting of a container (1) having a first chamber (fermentation chamber 3) for the hydrolysis and the acid fermentation and a second chamber (digestion chamber 4) separated by a partition (2) for the methane formation, with a line (5) for feeding a liquid substrate to the fermentation chamber (3) and a line (14) for discharging the treated substrate from the digestion chamber (4), with a gas discharge line (8, 13) for the resulting fermentation gas and digester gas and with a removal device (6) for the sludge forming in the bottom region of the fermentation chamber (3),
   characterised in that
   the line (5) for feeding substrate into the fermentation chamber (3) is located at a distance from the bottom of the fermentation chamber (3) at most at one third of the intended height of the liquid level in the fermentation chamber (3), that the connection between the fermentation chamber (3) and the digestion chamber (4) is designed as an overflow (9) which extends downwards into the digestion chamber (4) in the form of downward piping as far as at least one third of the height of the waste water discharge line (14), likewise designed as an overflow, and ends at a distance from the bottom of the digestion chamber (4) and that a forced circulation device and also a discharge pipe (12) in the bottom for the digested sludge are provided in the digestion chamber (4).

8.  A bioreactor according to Claim 7, characterised in that the forced circulation device is designed as a stirring device (10) with a vertically orientated tubular casing (11) surrounding it, which casing ends in each case at a distance from the reactor bottom and from the liquid level.

9.  A bioreactor according-to one of Claims 7 - 8, characterised in that the partition (2) separating the two chambers (3, 4) from one another is arranged such that the volume ratio of the fermentation chamber (3) to the digestion chamber (4) is between 1 : 1.5 and 1 : 5.

10.  A bioreactor according to one of Claims 7 - 9, characterised in that the forced circulation device is designed for an upward stream rate in the range of 0.8 to 1.3 m/h.

11.  A bioreactor according to one of Claims 7 - 10, characterised in that the substrate feed line (5) and the waste water discharge line (14) pass out of the wall of the reactor (1) close together and are integrated in the heat loss or heat absorption side of a heat exchanger.

12.  A bioreactor according to one of Claims 7 - 11, characterised in that the fermentation chamber (3) and the digestion chamber (4) each have a separate gas discharge line (8 and 13, respectively).

13.  A bioreactor according to one of Claims 7 - 12, characterised in that a layer of a bed (15) of solids particles with porous surfaces is arranged in the digestion chamber (4) above the mouth of the overflow (9).

14.  A bioreactor according to Claim 13, characterised in that the solids particles are made of ceramic.

15.  A bioreactor according to Claim 13 or 14, characterised in that the bed (15) is located on an intermediate plate designed as a sieve plate (16).

16.  A bioreactor according to one of Claims 13 - 15, characterised in that the bed (15) extends over the entire cross-section of the digestion chamber (4).

17.  A bioreactor according to Claim 10 and one of Claims 13 - 16, characterised in that the bed (15) is arranged about the tubular casing (11) of the stirring device (10).

## Revendications

1.  Procédé pour le traitement anaérobie, en deux étapes, de substrats liquides à forte charge organique dans un réacteur unique comportant deux chambres séparées par une paroi, l'hydrolyse et la fermentation acide ayant lieu dans la première chambre (chambre de fermentation) et la formation de méthane dans la seconde (chambre de putréfaction), des sédiments solides étant évacués à proximité du fond de la chambre de fermentation, l'introduction du substrat dans la chambre de putréfaction se faisant à partir de la partie supérieure de la chambre de fermentation, les eaux usagées générées par le substrat traité étant par ailleurs évacuées à la partie supérieure de la chambre de putréfaction, les gaz de fermentation et de putréfaction étant évacués au-dessus du niveau de liquide, caractérisé en ce que l'alimentation en substrat de la chambre de fermentation s'effectue à distance du fond de cette chambre, à une hauteur située au plus à un tiers du niveau du liquide dans cette chambre, que l'alimentation de la chambre de putréfaction avec un substrat débarrassé dans une large mesure de matières solides s'effectue à distance du fond de la chambre de putréfaction, à une hauteur au plus égale à un tiers du niveau du liquide dans cette chambre, que des sédiments solides sont évacués de la chambre de putréfaction et que le substrat est soumis à une circulation forcée dans la chambre de putréfaction.

2.  Procédé selon la revendication 1, caractérisé en ce que le courant ascendant dans la chambre de putréfaction est maintenu entre 0,8 et 1,3 m/h.

3.  Procédé selon une des revendications 1 ou 2, caractérisé en ce que le traitement des eaux usagées est effectué de façon continue.

4.  Procédé selon une des revendications 1 à 3, caractérisé en ce que le gaz de fermentation et le gaz de putréfaction sont collectés et évacués séparément.

5.  Procédé selon une des revendications 1 à 4, caractérisé en ce que l'on fait passer le substrat à travers un amas de granules solides dont la surface est peuplée de micro-organismes.

6.  Procédé selon la revendication 5, caractérisé en ce que l'on inverse de temps en temps la direction du courant du substrat dans la chambre de putréfaction.

7.  Réacteur biologique pour la mise en oeuvre du procédé selon la revendication 1, consistant en une enceinte (1) avec une première chambre (chambre de fermentation 3) pour l'hydrolyse et la fermentation acide, et avec une seconde chambre (chambre de putréfaction 4) séparée de la première par une paroi (2) pour la génération de méthane, avec une conduite d'alimentation (5) pour alimenter la chambre de fermentation (3) avec un substrat liquide et avec une conduite d'évacuation (14) pour évacuer le substrat traité de la chambre de putréfaction (4), avec une conduite d'évacuation de gaz (8, 13) pour le gaz de fermentation et le gaz de putréfaction produits, et avec un dispositif d'évacuation (6) pour la boue qui se forme à la partie basse de la chambre de fermentation (3), caractérisé en ce que l'entrée de la conduite d'alimentation en substrat (5) est située dans la chambre de fermentation (3) à distance du fond de celle-ci à une hauteur au plus égale à un tiers de la hauteur du niveau du liquide dans cette chambre, que le passage de la chambre de fermentation (3) à la chambre de putréfaction (4) est un trop-plein (9) en forme de tuyau s'étendant vers le bas dans la chambre de putréfaction (4) jusqu'à environ au moins un tiers de la hauteur de la conduite (14) d'évacuation des eaux usagées, laquelle forme également un trop-plein, et se termine à distance du fond de la chambre de putréfaction (4), que cette dernière contient un dispositif de circulation forcée, et qu'un nable de vidange (12) pour la boue de putréfaction est prévu dans le fond de la chambre de putréfaction (4).

8.  Réacteur biologique selon la revendication 7, caractérisé en ce que le dispositif de circulation forcée est réalisé sous forme d'un agitateur (10) entouré d'un tube vertical (11) dont les extrémités sont situées à distance du fond du réacteur ainsi que de la surface du liquide.

9.  Réacteur biologique selon une des revendications 7 ou 8, caractérisé en ce que la paroi (2) séparant les deux chambres (3, 4) est disposée de façon à ce que le rapport du volume de la chambre de fermentation (3) à celui de la chambre de putréfaction (4) soit compris entre 1 : 1,5 et 1 : 5.

10. Réacteur biologique selon une des revendications 7 à 9, caractérisé en ce que le dispositif de circulation forcée est dimensionné pour un courant ascendant compris entre 0,8 et 1,3 m/h.

11. Réacteur biologique selon une des revendications 7 à 10, caractérisé en ce que la conduite d'alimentation en substrat (5) et la conduite d'évacuation (14) des eaux usagées sortent côte à côte de l'enceinte (1) du réacteur et sont intégrées dans la partie réceptrice, respectivement distributrice de chaleur d'un échangeur de chaleur.

12. Réacteur biologique selon une des revendications 7 à 11, caractérisé en ce que tant la chambre de fermentation (3) que celle de putréfaction (4) comporte une conduite de gaz séparée (8 respectivement 13).

13. Réacteur biologique selon une des revendications 7 à 12, caractérisé en ce que la chambre de putréfaction (4) contient une couche formée d'un amas granuleux (15) de particules solides à surface poreuse.

14. Réacteur biologique selon la revendication 13, caractérisé en ce que les particules solides sont en céramique.

15. Réacteur biologique selon une des revendications 13 ou 14, caractérisé en ce que l'amas granuleux (15) est disposé sur un fond intermédiaire formant passoire (16).

16. Réacteur biologique selon une des revendications 13 à 15, caractérisé en ce que l'amas granuleux (15) s'étend sur toute la section transversale de la chambre de putréfaction (4).

17. Réacteur biologique selon la revendication 10 et une des revendications 13 à 16, caractérisé en ce que l'amas granuleux (15) est disposé autour du tube (11) entourant l'agitateur (10).

Fig. 1

Fig. 2